# EUROPÄISCHE PATENTSCHRIFT

(11) **EP 1 345 936 B1**
(45) Veröffentlichungstag und Bekanntmachung des Hinweises auf die Patenterteilung: **12.05.2004**
(21) Anmeldenummer: 01990546.2
(22) Anmeldetag: 12.12.2001
(51) Int. Cl.: C07D 451/10

(54) **VERFAHREN ZUR HERSTELLUNG DES ANTICHOLINERGIKUMS TIOTROPIUMBROMID**
METHOD FOR PRODUCING THE ANTICHOLINERGIC AGENT TIOTROPIUM BROMIDE
PROCEDE DE FABRICATION DE L'AGENT ANTICHOLINERGIQUE BROMURE DE TIOTROPIUM

(30) Priorität: 22.12.2000 DE 10064816
(43) Veröffentlichungstag der Anmeldung: 24.09.2003
(60) Teilanmeldung: 04005241.7
(73) Patentinhaber: Boehringer Ingelheim Pharma GmbH & Co.KG, 55216 Ingelheim am Rhein (DE)
(72) Erfinder: BANHOLZER, Rolf, 70597 Stuttgart (DE); GRAULICH, Manfred, 55425 Waldalgesheim (DE); LUETTKE, Sven, 55437 Ockenheim (DE); MATHES, Andreas, 55437 Ockenheim (DE); MEISSNER, Helmut, 55218 Ingelheim (DE); SPECHT, Peter, 55437 Ober-Hilbersheim (DE); BROEDER, Wolfgang, 55262 Heidesheim (DE)
(86) Internationale Anmeldenummer: PCT/EP2001/014566
(87) Internationale Veröffentlichungsnummer: WO 2002/051840

(56) Entgegenhaltungen:
- EP-A- 0 418 716
- PETROVIC, G. ET AL.: SYN. LETT., Bd. 5, 1999, Seiten 635-637, XP001068912

## Beschreibung

Die Erfindung betrifft ein neues Verfahren zur Herstellung von (1α,2β,4β,5α,7β)-7-[(Hydroxydi-2-thienylacetyl)oxy]-9,9-dimethyl-3-oxa-9-azoniatricyclo[3.3.1.0^{2,4}] nonanbromid.

### Hintergrund der Erfindung

Die Verbindung (1α,2β,4β,5α,7β)-7-[(Hydroxydi-2-thienylacetyl)oxy]-9,9-dimethyl-3-oxa-9-azoniatricyclo[3.3.1.0^{2,4}]nonan-bromid, ist aus der Europäischen Patentanmeldung EP 418 716 A1 bekannt und weist die folgende chemische Struktur auf:

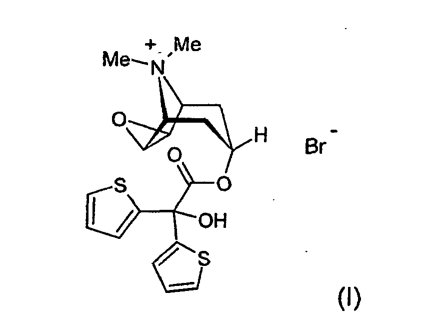

Die Verbindung besitzt wertvolle pharmakologische Eigenschaften und ist unter dem Namen Tiotropiumbromid (BA679) bekannt. Tiotropiumbromid stellt ein hochwirksames Anticholinergikum dar und kann deshalb bei der Therapie von Asthma oder COPD (chronic obstructive pulmonary disease = chronisch obstruktive Lungenerkrankung) einen therapeutischen Nutzen entfalten.

Die Applikation von Tiotropiumbromid erfolgt vorzugsweise auf inhalativem Wege. Hierbei können geeignete Inhaltionspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten.

Aufgrund seiner hohen Wirksamkeit ist Tiotropiumbromid schon in geringen therapeutischen Dosen einsetzbar. Dies stellt einerseits besondere Anforderungen an die pharmazeutische Herstellung der anzuwendenden Formulierung, andererseits ist es in besonderem Maße erforderlich, ein technisches Syntheseverfahren zur Herstellung von Tiotropiumbromid zu entwickeln, welches die Bereitstellung des Produkts nicht nur in guter Ausbeute sondern insbesondere in hervorragender Reinheit gewährleistet.

In der Europäischen Patentanmeldung EP 418 716 A1 wird ein synthetischer Zugang zu Tiotropiumbromid offenbart. Dieser entspricht der in Schema 1 skizzierten Vorgehensweise.

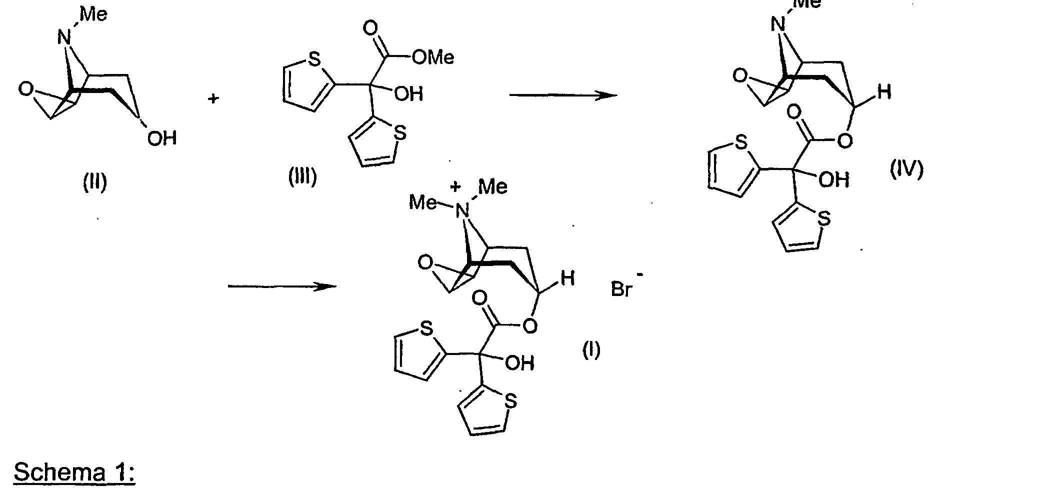

In einem ersten Schritt wird danach Scopin (II) mit Di-(2-thienyl)-glykolsäuremethylester (III) zum Di-(2-thienyl)-glykolsäurescopinester (IV) umgesetzt, welcher anschließend zum Tiotropiumbromid quarterniert wird.

### Detaillierte Beschreibung der Erfindung

Überraschenderweise wurde gefunden, daß Tiotropiumbromid in wesentlich größerer Reinheit erhalten werden kann, wenn die Synthese über einen anderen synthetischen Zugang als dem gemäß EP 418 716 A1 erfolgt. Dieser alternative und überraschenderweise vorteilhaftere Zugang ist schematisch in Schema 2 dargestellt.

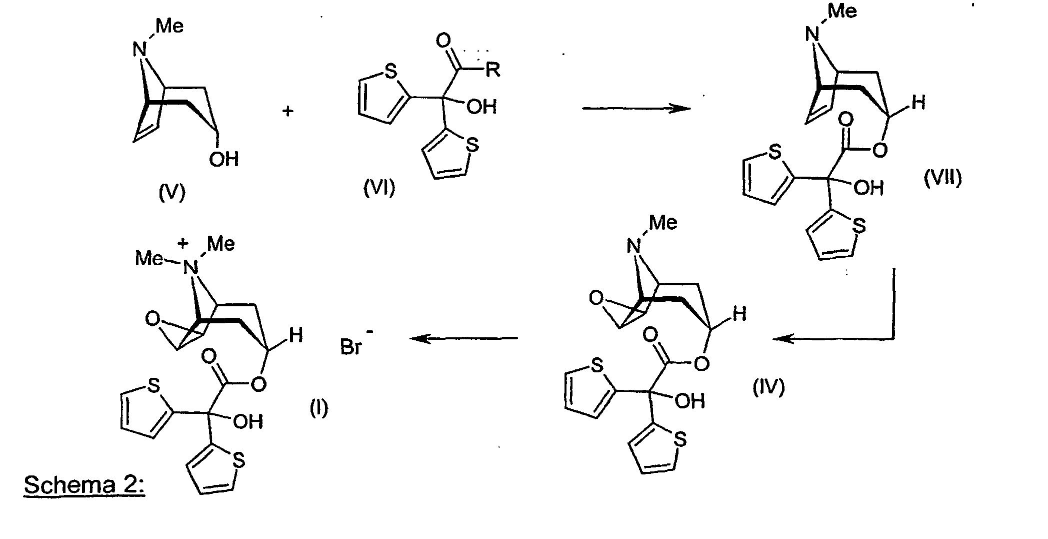

Ausgehend von im Stand der Technik bekannten Topenol (V) erfolgt durch Umsetzung mit Di-(2-thienyl)-glykolsäurederivaten (VI) zunächst die Bildung des Di-(2-thienyl)-glykolsäure-tropenolesters (VII). Dieser wird durch Epoxidation der olefinischen Doppelbindung in den entsprechenden Scopinester (IV) überführt.

Dementsprechend betrifft die vorliegende Erfindung ein Verfahren zur Herstellung von Tiotropiumpromid (I) 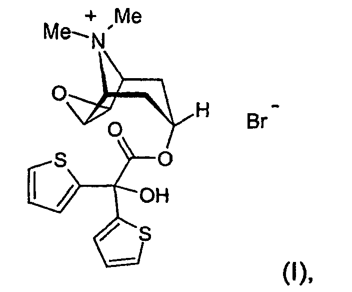 dadurch gekennzeichnet, daß der Tropenolester der Formel (VII) 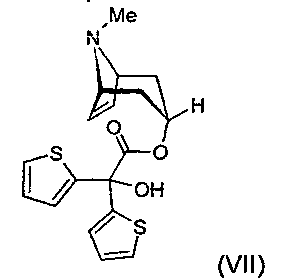 zum Scopinester der Formel (IV) 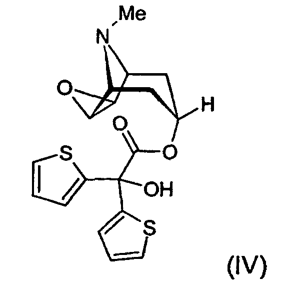 epoxidiert wird und dieser mittels Methylbromid zu Tiotropiumbromid (I) quartemiert wird.

Aufgrund der erfindungsgemäß zentralen Bedeutung des Tropenolestes der Formel (VII) betrifft ein weiterer Aspekt der vorliegenden Erfindung generell die Verwendung des Tropenolesters (VII), gegebenenfalls in Form seiner Säureadditionssalze, zur Herstellung von Tiotropiumbromid (I). Ein weiterer Aspekt der vorliegenden Erfindung betrifft ferner die Verwendung des Tropenolesters (VII), gegebenenfalls in Form seiner Säureadditionssalze, zur Herstellung des Scopinesters der Formel (IV).
Wird Tropenolester (VII) in Form eines Säureadditionssalzes zur Herstellung des Scopinesters (IV) eingesetzt, ist dieses Säureadditionssalz bevorzugt ausgewählt aus der Gruppe bestehend aus Hydrochlorid, Hydrobromid, Hydrogenphosphat, Hydrogensulfat, Tetrafluoroborat und Hexafluorophosphat, besonders bevorzugt sind das Hydrochlorid oder Hydrobromid.

Ein weiterer Aspekt der vorliegenden Erfindung betrifft ein Verfahren zur Herstellung von Tiotropiumbromid der Formel (I). 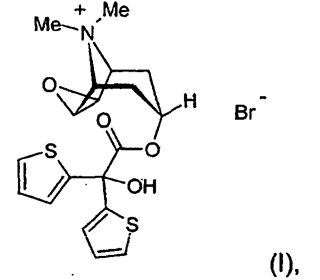 dadurch gekennzeichnet, daß in einem ersten Schritt Tropenol der Formel (V), 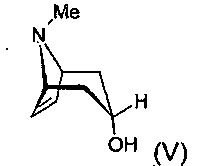 gegebenenfalls in Form seiner Säureadditionssalze, mit einem Ester der Formel (VI)

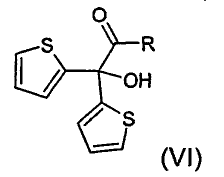

in dem R ein Rest ausgewählt aus der Gruppe bestehend aus Hydroxy, Methoxy, Ethoxy, O-N-Succinimid, O-N-Phthalimid, Phenyloxy, Nitrophenyloxy, Fluorophenyloxy, Pentafluorophenyloxy, Vinyloxy, -S-Methyl, -S-Ethyl und -S-Phenyl ist, zum Tropenolester der Formel (VII) 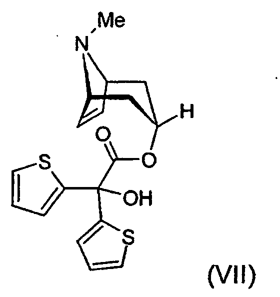 umgesetzt wird, dieser in einem zweiten Schritt zum Scopinester der Formel (IV) 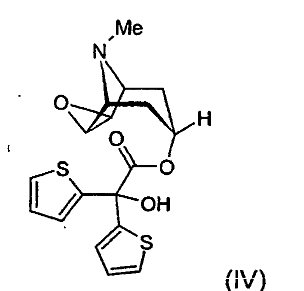 epoxidiert wird und dieser in einem dritten Schritt mittels Methylbromid zu Tiotropiumbromid (I) quarterniert wird.

Aufgrund der erfindungsgemäß zentralen Bedeutung des Tropenols (V) als Ausgangsmaterial zur Herstellung von Tiotropiumbromid (I) betrifft ein weiterer Aspekt der vorliegenden Erfindung ferner die Verwendung von Tropenol (V), gegebenenfalls in From seiner Säureaddtionssalze, als Ausgangsmaterial zur Herstellung von Tiotropiumbromid (I).

Zur Darstellung des Tropenolesters (VII) wird Tropenol, gegebenenfalls in Form eines Säureadditionssalzes ausgewählt aus der Gruppe bestehend aus Hydrochlorid, Hydrobromid, Hydrogenphosphat, Hydrogensulfat, Tetrafluoroborat und Hexafluorophosphat, vorzugsweise in Form des Hydrochlorids oder Hydrobromids, besonders bevorzugt in Form des Hydrochlorids in einem geeigneten organischen Lösemittel, vorzugsweise in einem Lösemittel ausgewählt aus der Gruppe bestehend aus Toluol, Benzol, n-Buthylacetat, Dichlormethan, THF, Dioxan, Dimethylacetamid, DMF und N-Methylpyrrolidinon, bevorzugt ausgewählt aus Toluol, Benzol, THF, Dioxan, Dimethylacetamid, DMF und N-Methylpyrrolidinon, besonders bevorzugt Toluol oder Benzol, wobei Toluol als Lösemittel höchstbevorzugt ist, aufgenommen. Pro Mol eingesetztes Tropenol (V) werden erfindungsgemäß 0,5 - 3 L, bevorzugt 0,75 - 2,5 L, besonders bevorzugt zwischen 1,25 und 1,75 L organisches Lösemittel eingesetzt.

In die so erhaltene Mischung wird, falls Tropenol in Form eines Säureadditionssalzes eingesetzt wird, eine Base zur Freisetzung des Tropenols zugesetzt. Als Base kommen erfindungsgemäß anorganische oder organische Basen in Betracht, wobei die Verwendung organischer Amine besonders bevorzugt ist. Als organische Amine können Triethylamin, Diisopropylethylamin, Pyridin, Dimethylaminopyridin , N-Methylpyrrolidin, N-Methylmorpholin oder Ammoniak verwendet werden, wobei die Verwendung von Triethylamin, Diisopropylethylamin, Pyridin oder Ammoniak bevorzugt, die von Ammoniak höchst bevorzugt ist. Pro Mol eingesetztes Tropenol-Salz werden wenigstens 1 Mol, vorzugsweise 1,25 bis 2,5 Mol, besonders bevorzugt 1,5 bis 2 Mol Amin zugesetzt. Die Zugabe des Amins kann bei Temperaturen zwischen 0 und 60°C, vorzugsweise 15 bis 50 °C, besonders bevorzugt 20 bis 30°C erfolgen. Nach beendeter Zugabe des Amins wird die erhaltene Suspension zwischen 0,1 bis 5 h, vorzugsweise zwischen 0,5 bis 2,5 h, besonders bevorzugt zwischen 0,75 und 1,5 h bei konstanter Temperatur gerührt.
Das dabei entstehende Ammoniumsalz wird abfiltriert und gegebenenfalls mit dem zuvor genannten organischen Lösemittel gewaschen. Pro Mol eingesetztes Tropenol (V) werden hierzu zwischen 0,1 und 1,5 L, bevorzugt 0,3 - 1,0 L Lösemittel verwendet.

Bei erhöhter Temperatur, vorzugsweise bei 30 - 80°C, besonders bevorzugt bei 40 bis 60°C wird im Vakuum ein Teil des Lösemittels abdestilliert. Die Destillationstemperatur ist naturgemäß abhängig von der Wahl des eingesetzten Lösemittels. Je nach Wahl des Lösemittels, wird das Vakuum so eingestellt, daß die Destillation im vorstehend genannten Temperaturbereich erfolgt. Pro Mol eingesetztes Tropenol (V) werden zwischen 0,25 und 2 L, bevorzugt zwischen 0,5 und 1,5 L Lösemittel abdestilliert. Nach Abdestillieren der vorstehend genannten Lösemittelmenge wird die Reaktionslösung auf einen Temperaturbereich von 0 - 50°C, vorzugsweise auf 15 - 35°C abgekühlt und das Di-(2-thienyl)glykolsäurederivat (VI) zugegeben. Als Di-(2-thienyl)glykolsäure-derivate (VI) kommen erfindungsgemäß solche Verbindungen in Betracht, in denen R Hydroxy, Methoxy, Ethoxy, O-N-Succinimid, O-N-Phthalimid, Phenyloxy, Nitrophenyloxy, Fluorophenyloxy, Pentafluorophenyloxy, Vinyloxy, -S-Methyl, -S-Ethyl oder -S-Phenyl bedeutet. Besonders bevorzugt wird die Verbindung (VI) eingesetzt, in der R für Hydroxy, Methoxy oder Ethoxy , besonders bevorzugt für Methoxy oder Hydroxy steht. Wird als Verbindung (VI) jene Verbindung gewählt, in der R Hydroxy bedeutet, kann die Umsetzung in Gegenwart von Kopplungsreagenzien wie Carbonyldiimidazol, Carbonyldi-1,2,4-triazol, Dicyclohexylcarbodiimid oder Ethyl-dimethylaminopropylcarbodiimid durchgeführt werden. Pro Mol eingesetztes Tropenol (V) werden zwischen 1 - 2 Mol der Verbindung (VI) verwendet. Bevorzugt werden 1 - 1,5 Mol (VI) eingesetzt, wobei der Einsatz stöchiometrischer Mengen von (VI) im Vergleich zu (V) erfindungsgemäß besonders bevorzugt ist. Gegebenenfalls wird die erhaltene Reaktionsmischung zur Herstellung einer Lösung erwärmt. Dabei wird eine Temperatur im Bereich von 30 - 80°C, bevorzugt von 40 - 60°C, besonders bevorzugt von etwa 45-55°C gewählt.

Die so erhaltene Lösung wird dann zu einer weiteren Lösung oder Mischung einer anorganischen oder organischen Base in einem der vorstehend genannten Lösemittel, vorzugsweise in dem Lösemittel, welches zur Herstellung der Mischung aus (V) und (VI) Verwendung findet, gegeben. Pro Mol eingesetztes Tropenol (V) werden zur Herstellung der Basen-haltigen Lösung oder Mischung zwischen 0,2 und 2,0 L, bevorzugt 0,4 - 1,5 L, besonders bevorzugt 0,5 bis 1,0 L Lösemittel verwendet. Im Fall von R gleich Methoxy, Ethoxy, Vinyloxy, Phenyloxy, -S-Methyl, -S-Ethyl oder -S-Phenyl erfolgt die Umsetzung in Gegenwart einer organischen oder anorganischen Base. Als organische Base kommen bevorzugt organische Amine, besonders bevorzugt Diisopropylethylamin, Triethylamin, cyclische Amine wie DBU, oder Pyridin in Betracht. Als anorganische Base kommen die Alkali- oder die Erdalkalicarbonate, die Alkoholate und Hydride des Lithiums, Natriums, Kaliums, Calciums wie Natriumcarbonat, Lithiumcarbonat, Kaliumcarbonat, Calciumcarbonat, Natriumhydrid, Kaliumhydrid, Calciumhydrid, Natriummethylat, Natriumethylat, Kaliummethylat oder Kaliumethylat in Betracht. Besonders bevorzugt wird als anorganische Base eines der vorstehend genannten Hydride oder Alkoholate, bevorzugt eines der genannten Hydride, eingesetzt, wobei die Verwendung von Natriumhydrid erfindungsgemäß besonders bevorzugt ist. Pro Mol Tropenol (V) werden wenigstens stöchiometrische Mengen an Base eingesetzt. Bevorzugt werden pro Mol Tropenol (V) 1 - 3 Mol, besonders bevorzugt 1,25 - 2,5 Mol, höchst bevorzugt 1,5 bis 2 Mol Base eingesetzt.

Die Lösung von (V) und (VI) wird mit der vorstehend beschriebenen Basen-haltigen Lösung oder Mischung vorzugsweise über einen Zeitraum von 0,2 - 2,0 h, bevorzugt über einen Zeitraum von 0,5 bis 1,5 h versetzt. Wird als Verbindung (VI) beispielsweise eine Ester eingesetzt, in dem R Methoxy oder Ethoxy bedeutet, kann es erforderlich sein, bei 40-90°C, bevorzugt bei 50 bis 80°C, besonders bevorzugt bei 60-75°C unter Vakuum, bevorzugt bei 150 bis 500 mbar, besonders bevorzugt bei 200 - 350 mbar, höchst bevorzugt bei 250 - 300mbar den entstehenden Alkohol abzudestillieren. Durch eine solche Vorgehensweise wird das Reaktionsgleichgewicht auf die Seite des Tropenolesters (VII) verschoben. Unter diesen Reaktionsbedingungen wird auch ein Teil des Lösemittels mit abdestilliert.

Nach beendeter Destillation (ca. 5 bis 10 h), kann die Menge an abdestilliertem Lösemittel der Reaktionslösung gegebenenfalls wieder zugeführt werden.
In jedem Fall wird die erhalten Lösung nach beendeter Destillation wieder abgekühlt auf einen Temperaturbereich von unter 40°C, vorzugsweise auf 0 - 35°C, besonders bevorzugt auf 10 - 25°C.
Zu dieser Mischung wird bei gleichbleibender Temperatur über einen Zeitraum von 0,2 bis 2 h, vorzugsweise 0,4 - 0,6 h Salzsäure gegeben. Die Zugabe der Salzsäure kann entweder in Form wässriger Lösungen oder gasförmig erfolgen, wobei die Zugabe wässriger Lösungen bevorzugt ist. Vorzugsweise wird konzentrierte Salzsäure (36%-ig) in Wasser gelöst zugegeben. Pro Mol eingesetztes Tropenol (V) werden vorzugsweise zwischen 1 und 4 Mol, bevorzugt 1,5 - 3 Mol, besonders bevorzugt 2,0 bis 2,5 Mol HCl zugegeben. Bevorzugt werden pro Mol Tropenol (V) 0,1 - 0,4 kg, besonders bevorzugt 0, 15 - 0,25 kg 36%-ige wässrige Salzsäure in 10 - 20 L, bevorzugt in 12 - 17 L Wasser gelöst zugesetzt.

Nach beendeter Zugabe und guter Durchmischung des Ansatzes wird die wässrige Phase abgetrennt. Diese wird mit einem geeigneten, mit Wasser nicht mischbaren organischen Lösemittel gewaschen. Bevorzugt wird hierfür ein mit Wasser nicht mischbares Lösemittel ausgewählt aus der Gruppe bestehend aus Methylenchlorid und n-Butylacetat, bevorzugt Methylenchlorid verwendet. Gegebenenfalls wird die erste zur Extraktion der wässrigen Phase verwendete organische Phase verworfen und der Extraktionsvorgang ein weiteres mal durchgeführt.

Die wässrige Phase, gegebenenfalls nach vorherigem Waschen mit einem der vorstehend genannten, nicht mit Wasser mischbaren Lösemittel, wird ein weiteres mal mit dem nicht mit Wasser mischbaren Lösemittel versetzt. Vorzugsweise werden pro Mol ursprünglich eingesetzten Tropenol (V) 1 - 5 L, bevorzugt 2 - 4 L, besonders bevorzugt 2,5 - 3,5 L des nicht mit Wasser mischbaren Lösemittels verwendet. Die so erhaltene Mischung wird mit einer anorganischen Base, vorzugsweise ausgewählt aus den Alkali- oder Erdalkalicarbonaten des Lithiums, Natriums, Kaliums, Calciums wie beispielsweise Natriumcarbonat, Lithiumcarbonat, Kaliumcarbonat oder Calciumcarbonat, wobei Natriumcarbonat besonders bevorzugt ist, versetzt und damit auf einen pH von 7,5 bis 11, bevorzugt 8 bis 10, eingestellt. Die Zugabe der anorganischen Base erfolgt vorzugsweise in Form wässriger Lösungen. Beispielsweise und erfindungsgemäß besonders bevorzugt werden pro Mol eingesetztes Tropenol (V) 0,05 bis 0,4 kg, bevorzugt 0,1 bis 0,2 kg anorganische Base in 0,25 bis 1,5 L, bevorzugt in 0,5 bis 1 L, besonders bevorzugt in 0,7 bis 0,8 L Wasser gelöst zugegeben.

Nach guter Durchmischung des erhaltenen Reaktionsgemischs wird die wässrige Phase abgetrennt und ein- oder mehrmals mit dem zuvor genannten nicht mit Wasser mischbaren Lösemittel extrahiert. Pro Mol ursprünglich eingesetztes Tropenol (V) werden zur Extraktion der wässrigen Phase insgesamt 1 - 8 L, bevorzugt 2 - 6 L, besonders bevorzugt 3 - 5 L des zuvor genannten, nicht mit Wasser mischbaren Lösemittels verwendet. Die vereinigten organischen Phasen werden anschließend bei erhöhter Temperatur, vorzugsweise bei 30 - 90°C, besonders bevorzugt bei 50 - 70°C destillativ vom Lösemittel befreit. Die vorstehend genannten Temperaturbereiche sind, wie für den Fachmann einsichtig, stark von der Wahl der verwendeten Lösemittels abhängig. Gegebenenfalls kann zu dieser destillativen Lösemittel-Entfernung auch ein Vakuum angelegt werden, um die Temperatur in den zuvor definierten Temperaturbereichen zu halten: Maximale Destillationstemperatur ist bei Lösemitteln, die unterhalb der vorstehend definierten maximalen Temperturbereiche abdestillieren, naturgemäß der Siedepunkt des jeweiligen Lösemittels.

Der nach der Destillation verbleibende Rückstand wird in einem organischen Lösemittel aufgenommen. Dieses Lösemittel kann ausgewählt werden aus der Gruppe der Lösemittel, die gemäß der vorliegenden Beschreibung zur Durchführung der Umsetzung von (V) und (VI) zu (VII) verwendet werden können. Vorzugsweise wird dasselbe Lösemittel verwendet, was bei dieser Umsetzung zum Einsatz gelangt. Pro Mol ursprünglich eingesetzten Tropenols (V) werden zur Lösung des Rückstands 1 - 5 L, vorzugsweise 1,5 - 4 L, bevorzugt 2 - 3 L Lösemittel verwendet. Die so erhaltene Lösung wird erwärmt, maximal bis auf Siedetemperatur des Lösemittels, bevorzugt auf einen Bereich von 50 - 100°C, besonders bevorzugt auf 80 - 95°C. Die erwärmte Lösung wird langsam auf eine Temperatur im Bereich von -10°C bis 20°C abgekühlt, bevorzugt auf 0 - 10°C. Der Tropenolester (VII) fällt in Form farbloser Kristalle an, die abgetrennt und getrocknet werden. Die Trocknung erfolgt vorzugsweise unter Inertgas bei Temperaturen von 30 - 50°C.

Der so erhaltene Tropenolester (VII) wird nun, wie nacholgend beschrieben, zum Scopinester (IV) epoxidiert. In einem geeigneten Reaktionsapparat wird ein geeignetes Lösemittel, bevorzugt ausgewählt aus der Gruppe bestehend aus Wasser, Dimethylformamid, Acetonitril, Dimethylacetamid und N-Methylpyrrolidinon, besonders bevorzugt Dimethylformamid vorgelegt und auf eine Temperatur im Bereich von 30 - 70°C, bevorzugt 40 - 60°C erwärmt. Pro Mol eingesetzten Tropenolester (VII) werden 2 - 10 L, vorzugsweise 3 - 8 L, bevorzugt 4 - 7 L, besonders bevorzugt 5 - 6 L Lösemittel verwendet. In das wie vorstehend beschrieben erwärmte Lösemittel wird der Tropenolester (VII) eingetragen und die erhaltene Mischung solange bei konstanter Temperatur gerührt, bis eine klare Lösung erhalten worden ist.

Anschließend wird zu dieser Lösung bei einer Temperatur im Bereich von 20 - 50°C, bevorzugt bei 35 - 45°C portionsweise ein Epoxidierungsmittel eingetragen. Als Epoxidierungsmittel kommt bevorzugt Vanadiumpentoxid im Gemisch mit H₂O₂, besonders bevorzugt H₂O₂-Harnstoffkomplex in Kombination mit Vanadiumpentoxid .zur Anwendung. Bevorzugt erfolgt die Zugabe von Wasserstoffperoxid-Hamstoffkomplex und Vanadiumpentoxid portionsweise im Wechsel, besonders bevorzugt wird ferner Wasser zugesetzt. Pro Mol eingesetzten Tropenolester (VII) werden 0,1 - 0,5 kg, bevorzugt 0,15 - 0,3 kg Wasserstoffperoxid-Hamstoffkomplex, 0,1 - 1,0 L, bevorzugt 0,15 - 0,7 L, besonders bevorzugt 0,2-0,4 L Wasser sowie 0,001 - 0,1 kg, bevorzugt 0,005 - 0,05 kg, besonders bevorzugt 0,01 - 0,025 kg Vanadiumpentoxid eingesetzt. Nach beendeter Zugabe wird für einen Zeitraum von 1 - 6 h, vorzugsweise 1,5 - 4 h, bevorzugt 2 - 3 h bei einer Temperatur von 30 - 70°C, bevorzugt 40 -60°C, besonders bevorzugt 45 -55°C gerührt.

Anschließend wird auf eine Temperatur im Bereich von 10 - 30°C, vorzugsweise auf 15 - 25°C abgekühlt und mit Salzsäure ein pH von 2,5 - 5,5, vorzugsweise ein pH von 3,5 - 4,5 eingestellt. Die Zugabe der Salzsäure kann entweder in Form wässriger Lösungen oder gasförmig erfolgen, wobei die Zugabe wässriger Lösungen bevorzugt ist. Vorzugsweise wird konzentrierte Salzsäure (36%-ig) in Wasser gelöst zugegeben. Nach guter Durchmischung erfolgt die Zugabe eines anorganischen Salzes, bevorzugt Natriumhydrogensulfit. Bevorzugt wird letzteres in Form wässriger Lösungen zugegeben. Besonders bevorzugt werden pro Mol eingesetzten Tropenolester (VII) 20 - 100g, vorzugsweise 30 - 80 g, besonders bevorzugt 40 -60 g anorganisches Salz gelöst in 0,1 - 1 L, bevorzugt 0,3 - 0,7 L Wasser (jeweils pro Mol eingesetzte Verbindung (VII)) zugegeben. Bei einer Innentemperatur von 20 - 50°C, bevorzugt 30 - 40°C wird ein Teil des Lösemittels abdestilliert. Pro Mol eingesetzte Verbindung werden etwa 2 - 8 L, bevorzugt 3 - 6 L des Lösemittels entfernt. Nach Abkühlen auf etwa 15-25°C wird mit Clarcel (Celite) versetzt (pro Mol eingesetzte Verbindung (VII) etwa 40 -100 g, bevorzugt 60 - 80 g). Durch erneute Zugabe von Salzsäure, bevorzugt von verdünnter wässriger Salzsäure, wird ein pH von 1-3, bevorzugt von 1,5 - 2,5 eingestellt. Pro Mol eingesetzte Verbindung (VII) werden vorzugsweise 10 - 30g, bevorzugt 15 - 20 g 36%-ige Salzsäure, gelöst in 5 - 15 L, bevorzugt 8 - 12 L Wasser (pro Mol eingesetztes (VII)) verwendet.

Die erhaltene Lösung wird filtriert und gegebenenfalls mit einem geeigneten, mit Wasser nicht mischbaren Lösemittel ein-, zwei- oder dreimal extrahiert. Bevorzugt wird hierfür ein mit Wasser nicht mischbares Lösemittel ausgewählt aus der Gruppe bestehend aus Methylenchlorid und n-Butylacetat, bevorzugt Methylenchlorid verwendet. Die zur Extraktion der wässrigen Phase verwendeten organischen Phasen werden verworfen.
Die wässrige Phase, gegebenenfalls nach vorherigem Waschen mit einem der vorstehend genannten, nicht mit Wasser mischbaren Lösemittel, wird ein weiteres mal mit dem nicht mit Wasser mischbaren Lösemittel versetzt. Vorzugsweise werden pro Mol ursprünglich eingesetzten Tropenolesters (VII) 1 - 5 L , bevorzugt 2 - 4 L, besonders bevorzugt 2,5 - 3,5 L des nicht mit Wasser mischbaren Lösemittels verwendet. Die so erhaltene Mischung wird mit einer anorganischen Base, vorzugsweise ausgewählt aus den Alkali- oder Erdalkalicarbonaten des Lithiums, Natriums, Kaliums, Calciums wie beispielsweise Natriumcarbonat, Lithiumcarbonat, Kaliumcarbonat oder Calciumcarbonat, wobei Natriumcarbonat besonders bevorzugt ist, versetzt und damit auf einen pH von 8 bis 11, bevorzugt 9 bis 10,5 eingestellt. Die Zugabe der anorganischen Base erfolgt vorzugsweise in Form wässriger Lösungen. Beispielsweise und erfindungsgemäß besonders bevorzugt werden pro Moi eingesetzten Ester (VII) 0,05 bis 0,4 kg, bevorzugt 0,15 bis 0,3 kg Natriumcarbonat in 0,25 bis 2 L, bevorzugt in 0,75 bis 1,25 L gelöst zugegeben.

Nach guter Durchmischung des erhaltenen Reaktionsgemischs wird die wässrige Phase abgetrennt und ein- oder mehrmals mit dem zuvor genannten nicht mit Wasser mischbaren Lösemittel extrahiert. Pro Mol ursprünglich eingesetzten Tropenolester (VII) werden zur Extraktion der wässrigen Phase insgesamt 1 - 5 L, bevorzugt 2 - 4 L des zuvor genannten, nicht mit Wasser mischbaren Lösemittels verwendet. Die vereinigten organischen Phasen werden anschließend bei vorzugsweise 25 - 50°C, besonders bevorzugt bei 30 - 40°C destillativ teilweise vom Lösemittel befreit. Die vorstehend genannten Temperaturbereiche sind, wie für den Fachmann einsichtig, stark von der Wahl der verwendeten Lösemittels abhängig. Gegebenenfalls kann zu dieser destillativen Lösemittel-Entfernung auch ein Vakuum angelegt werden, um die Temperatur in den zuvor definierten Temperaturbereichen zu halten. Vorzugsweise wird im schwachen Vakuum bei 500 - 800 mbar, bevorzugt bei 600-700 mbar abdestilliert. Pro Mol ursprünglich eingesetzten Ester (VII) werden etwa 2 - 6 L, bevorzugt 3 - 5 L des Lösemittels abdestilliert.

Gegebenenfalls kann es erforderlich sein, Verunreinigungen in Form sekundärer Amine an dieser Stelle zu entfernen. Dies erfolgt erfindungsgemäß durch Verwendung organische Carbonsäurehalogenide, vorzugsweise durch Säurechloride ausgewählt aus der Gruppe bestehend aus Acetylchlorid, Propionsäurechlorid oder Buttersäurechlorid. Vorzugsweise wird Acetylchlorid verwendet. Üblicherweise werden pro Mol ursprünglich eingestzten Esters (VII) zwischen 5 und 30 g, vorzugsweise 10 - 20 g Carbonsäurehalogenid eingesetzt. Nach Zugabe des Carbonsäurehalogenids bei 15-25°C wird 15 Minuten bis 1,5 h, vorzugsweise zwischen 30 und 45 Minuten bei gleichbleibender Temperatur gerührt. Anschließend wird auf eine Temperatur im Bereich von 10 - 30°C, vorzugsweise auf 15 - 25°C eingestellt und mit Salzsäure ein pH von 1 - 3, vorzugsweise ein pH von 1,5 - 2,5 eingestellt. Die Zugabe der Salzsäure kann entweder in Form wässriger Lösungen oder gasförmig erfolgen, wobei die Zugabe wässriger Lösungen bevorzugt ist. Vorzugsweise wird konzentrierte Salzsäure (36%-ig) in Wasser gelöst zugegeben. Pro Mol eingesetzte Verbindung (VII) werden vorzugsweise 0,05 - 0,5kg, bevorzugt 0,075 - 1,25kg 36%-ige Salzsäure, gelöst in 5 -15 L, bevorzugt 8 -12 L Wasser (pro Mol eingesetztes (VII)) verwendet. Die organische Phase wird abgetrennt und entsorgt.

Die wässrige Phase, gegebenenfalls nach vorherigem Waschen mit einem der vorstehend genannten, nicht mit Wasser mischbaren Lösemittel, wird ein weiteres mal mit dem nicht mit Wasser mischbaren Lösemittel versetzt. Vorzugsweise werden pro Mol ursprünglich eingesetzten Tropenolesters (VII) 1 - 5 L , bevorzugt 2 - 4 L, besonders bevorzugt 2,5 - 3,5 L des nicht mit Wasser mischbaren Lösemittels verwendet. Die so erhaltene Mischung wird mit einer anorganischen Base, vorzugsweise ausgewählt aus den Alkali- oder Erdalkalicarbonaten des Lithiums, Natriums, Kaliums, Calciums wie beispielsweise Natriumcarbonat, Lithiumcarbonat, Kaliumcarbonat oder Calciumcarbonat, wobei Natriumcarbonat besonders bevorzugt ist, versetzt und damit auf einen pH von 8 bis 11, bevorzugt 9 bis 10,5 eingestellt. Die Zugabe der anorganischen Base erfolgt vorzugsweise in Form wässriger Lösungen. Beispielsweise und erfindungsgemäß besonders bevorzugt werden pro Mol eingesetzten Ester (VII) 0,05 bis 0,4 kg, bevorzugt 0,1 bis 0,2 kg Natriumcarbonat in 0,25 bis 2 L, bevorzugt in 0,7 bis 1,2 L gelöst zugegeben.

Nach guter Durchmischung des erhaltenen Reaktionsgemischs wird die wässrige Phase abgetrennt und ein - oder bevorzugt zweimal mit dem zuvor genannten nicht mit Wasser mischbaren Lösemittel extrahiert. Pro Mol ursprünglich eingesetzten Tropenolester (VII) werden zur Extraktion der wässrigen Phase 0,5 - 2,5 L, bevorzugt 1 - 2 L des zuvor genannten, nicht mit Wasser mischbaren Lösemittels verwendet. Die vereinigten organischen Phasen werden anschließend bei vorzugsweise 25 - 50°C, besonders bevorzugt bei 30 - 40°C destillativ teilweise vom Lösemittel befreit (pro Mol eingesetzten Ester (VII) werden etwa 1-3 L, bevorzugt 1,5 - 2,5 L Lösemittel entfernt). Es wird anschließend ein Lösemittels ausgewählt aus Dimethylformamid, Dimethylacetamid, N-Methylpyrrolidinon oder Dichlormethan, bevorzugt Dimethylformamid zugegeben. Pro Mol eingesetzten Ester (VII) werden zwischen 1 und 5 kg, bevorzugt zwischen 1,5 und 4 kg, besonders bevorzugt zwischen 2 und 3 kg Lösemittel verwendet. Aus dieser Lösung werden bei schwachem Vakuum (600-700mbar) und einer Temperatur von 30-40°C die verbliebenen Reste des zuvor zur Extraktion verwendeten mit Wasser nicht mischbaren Lösemittels abdestilliert.
Die so erhaltene Lösung an Scopinester (IV) wird ohne weitergehende Isolierung der Zwischenverbindung direkt in die nächste Stufe eingesetzt.

Zur Herstellung von Tiotropiumbromid (I) wird in die nach vorhergehender Vorschrift erhältliche Scopinester-Lösung bei 10-30°C, bevorzugt bei 15-25°C Methylbromid eingeleitet. Da in diese Stufe eine Lösung von Scopinester (IV) eingesetzt wird, ohne daß eine Bestimmung der Ausbeute der vorhergehenden Stufe erfolgt, werden die nachfolgenden Mengenangaben auf ursprünglich eingesetzten Tropenolester (VII) bezogen. Pro Mol eingesetzten Scopinester (IV) wird zumindest 1 Mol Methylbromid verwendet. Erfindungsgemäß bevorzugt werden pro Mol eingesetzten Tropenolester (VII) 0,1 - 0,2 kg, bevorzugt 0,11 - 0,15 kg Methylbromid verwendet. Nach beendeter Zugabe des Methylbromids wird entweder bei 15-35°C für 1-3 Tage, bevorzugt für 48 - 72 Stunden gerührt. Im Vakuum wird anschließend bei 30 - 60 °C, vorzugsweise bei 45 -55°C das Lösemittel Dimethylformamid teilweise abdestilliert. Das Vakuum wird so gewählt, daß ein Abdestillieren des Lösemittels in den vorstehend genannten Temperaturbereichen erfolgt. Pro Mol eingesetzten Tropenolester (VII) werden etwa. 0,5 - 2,0 L, bevorzugt 1,0-1,75 L Lösemittel abdestilliert und anschließend auf etwa 5-20°C, bevorzugt 10-15°C abgekühlt. Bei dieser Temperatur wird bis zur vollständigen Kristallisation des Rohprodukts nachgerührt und die ausgefallenen Kristalle abgetrennt und bei 30-50°C unter Inertgas, vorzugsweise Stickstoff, getrocknet.

Eine weitergehende Reinigung des Produkts kann durch Kristallisation aus Methanol erfolgen. Pro 1 Mol Tiotropiumbromid (I) werden etwa 2 - 8 L, bevorzugt 3 - 7 L, besonders bevorzugt 4 - 5 L Methanol verwendet und das so erhaltene Gemisch bis zur Lösung des Produkts zum Rückfluß erhitzt. Anschließend wird auf 0-15°C, bevorzugt 3-7°C abgekühlt und unter Rühren das Produkt kristallisiert. Nach vollständiger Kristallisation werden die Kristalle abgetrennt, gegebenenfalls mit kaltem Methanol gewaschen und abschließend bei 30-50°C unter Inertgas, vorzugsweise Stickstoff, getrocknet.

Gegebenenfalls kann das so erhaltene Produkt in sein Monohydrat überführt werden. Hierzu kann wie nachfolgend beschrieben vorgegangen werden.

In einem geeignet dimensionierten Reaktionsgefäß wird das Lösemittel mit Tiotropiumbromid, gemischt. Pro Mol eingesetztes Tiotropiumbromid werden 0,4 bis 1,5 kg, bevorzugt 0,6 bis 1 kg, besonders bevorzugt ca. 0,8 kg Wasser als Lösemittel verwendet. Die erhaltene Mischung wird unter Rühren erwärmt, vorzugsweise auf mehr als 50°C, besonders bevorzugt auf mehr als 60°C. Die maximal wählbare Temperatur bestimmt sich durch den Siedepunkt des verwendeten Lösemittels Wasser. Vorzugsweise wird die Mischung auf einen Bereich von 80-90°C erhitzt.
In diese Lösung wird Aktivkohle, trocken oder wasserfeucht, eingebracht. Bevorzugt werden pro Mol eingesetztes Tiotropiumbromid 10 bis 50 g, besonders bevorzugt 15 bis 35 g, höchst bevorzugt etwa 25 g Aktivkohle eingesetzt. Gegebenenfalls wird die Aktivkohle vor Einbringen in die Tiotropiumbromid-haltige Lösung in Wasser aufgeschlämmt. Pro Mol eingesetztes Tiotropiumbromid werden zum Aufschlämmen der Aktivkohle 70 bis 200 g, bevorzugt 100 bis 160 g, besonders bevorzugt ca. 135 g Wasser verwendet. Wird die Aktivkohle vor Einbringen in die Tiotropiumbromid-haltige Lösung zuvor in Wasser aufgeschlämmt, empfiehlt es sich, mit der gleichen Menge Wasser nachzuspülen.
Bei konstanter Temperatur wird nach erfolgter Aktivkohlezugabe zwischen 5 bis 60 Minuten, bevorzugt zwischen 10 und 30 Minuten, besonders bevorzugt etwa 15 Minuten weitergerührt und die erhaltene Mischung filtriert, um die Aktivkohle zu entfernen. Der Filter wird anschließend mit Wasser nachgespült. Hierfür werden pro Mol eingesetztes Tiotropiumbromid 140 bis 400 g, bevorzugt 200 bis 320 g, höchst bevorzugt ca. 270 g Wasser verwendet.
Das Filtrat wird anschließend langsam abgekühlt, vorzugsweise auf eine Temperatur von 20-25°C. Die Abkühlung wird vorzugsweise mit einer Abklühlrate von 1 bis 10°C pro 10 bis 30 Minuten, bevorzugt von 2 bis 8°C pro 10 bis 30 Minuten, besonders bevorzugt von 3 bis 5°C pro 10 bis 20 Minuten, höchst bevorzugt von 3 bis 5°C pro ca. 20 Minuten durchgeführt. Gegebenenfalls kann sich nach dem Abkühlen auf 20 bis 25°C eine weitere Abkühlung auf unter 20°C, besonders bevorzugt auf 10 bis 15°C anschließen.
Nach erfolgter Abkühlung wird zwischen 20 Minuten und 3 Stunden, vorzugsweise zwischen 40 Minuten und 2 Stunden, besonders bevorzugt etwa eine Stunde zur Vervollständigung der Kristallisation nachgerührt.
Die entstandenen Kristalle werden abschließend durch Filtrieren oder Absaugen des Lösemittels isoliert. Sollte es erforderlich sein, die erhaltenen Kristalle einem weiteren Waschschritt zu unterwerfen, empfiehlt es sich als Waschlösemittel Wasser oder Aceton zu verwenden. Pro Mol eingesetztes Tiotropiumbromid können zum Waschen der erhaltenen Tiotropiumbromid-monohydrat-Kristalle 0,1 bis 1,0 L, bevorzugt 0,2 bis 0,5 L, besonders bevorzugt etwa 0,3 L Lösemittel Verwendung finden. Gegebenenfalls kann der Waschschritt wiederholt durchgeführt werden.

Das erhaltene Produkt wird im Vakuum oder mittels erwärmter Umluft bis zum Erreichen eines Wassergehalts von 2,5 - 4,0 % getrocknet.

Die nachfolgenden Beispiele dienen der illustration exemplarisch durchgeführter Syntheseverfahren zur Herstellung von Tiotropiumbromid. Sie sind lediglich als mögliche, exemplarisch dargestellte Vorgehensweisen zu verstehen, ohne die Erfindung auf deren Inhalt zu beschränken.

### Darstellung des Tropenolesters (VII)

Zu 10,9 kg Tropenolhydrochlorid in Toluol (95 L) wird bei 25°C Ammoniak (1,8 kg) eingeleitet. Die erhaltene Suspension wird bei konstanter Temperatur etwa 1 h gerührt. Anschließend wird entstandenes Ammoniumhydrochlorid abfiltriert und mit Toluol (26 L) nachgespült. Bei etwa 50°C Manteltemperatur wird ein Teil des Toluols (ca 60 L) im Vakuum abdestilliert. Nach Abkühlen auf etwa 25°C werden 15,8 kg Di-(2-thienyl)glykolsäuremethylester zugegeben und die erhaltene Mischung zum Lösen auf 50°C erwärmt. In einem weiteren Apparat wird Toluol (40 L) vorgelegt und in diese bei etwa 25°C Natriumhydrid (2,7 kg) eingetragen. in diese Lösung wird innerhalb von 1 h die zuvor generierte Lösung aus Tropenol und Glykolsäuremethylester bei 30°C gegeben. Nach beendeter Zugabe wird bei reduziertem Druck etwa 7 Stunden unter Rühren auf 75°C erhitzt. Das sich bildende Methanol und wird dabei abdestilliert. Die verbleibende Mischung wird abgekühlt und in eine Mischung von Wasser (958 L) und 36 %iger Salzsäure (13,2 kg) gegeben. Die wäßrige Phase wird anschließend abgetrennt und mit Methylenchlorid (56 L) gewaschen. Nach erneuter Methylenchloridzugabe (198 L) wird das so erhaltene Gemisch mit vorbereiteter Sodalösung (9,6 kg Soda in 45 L Wasser) auf pH = 9 gestellt. Die Methylenchlorid-Phase wird abgetrennt und die wässrige Phase mit Methylenchlorid (262 L) ausgerührt. Die Methylenchlorid-Phasen werden bei 65°C bis auf den Rückstand eingeengt. Den Rückstand wird in Toluol (166 L) aufgenommen und auf 95°C erhitzt. Die toluolische Lösung wird auf 0°C abgekühlt. Die erhaltenen Kristalle werden abgetrennt, mit Toluol (33 L) gewaschen und bei etwa 50°C für max. 24 Std. im Stickstoffstrom getrocknet.
Ausbeute: 18,6 kg (83%);Schmp.: ca. 160°C (bestimmt über DSC bei Heizrate von 10K/min);

### Darstellung des Scopinesters (IV)

In einem geeigneten Reaktionsapparat werden 260 L DMF vorgelegt und auf 50°C erwärmt. Anschließend werden 16,2 kg Tropenolester (VII) zugegeben und es wird gerührt, bis eine klare Lösung erhalten worden ist. Nach Abkühlen auf 40°C werden nacheinander und portionsweise Wasserstoffperoxid-Harnstoff-Komplex (10,2 kg), Wasser (13 L) und Vanadium-(V)-oxid (0,7 kg) eingetragen und der Apparateinhalt auf etwa 50°C erhitzt. Nach 2 - 3 h Rühren bei konstanter Temperatur wird etwa 20°C abgekühlt. Das erhaltene Reaktionsgemisch wird mit Salzsäure (36 %ig) auf etwa pH 4,0 eingestellt. Vorbereitete Natriumbisulfit-Lösung (2,4 kg in 24 L Wasser) wird zugegeben. Bei einer Innentemperatur von 35°C wird das Lösemittel im Vakuum teilweise abdestilliert (etwa 210 L). Es wird wiederum auf etwa 20°C abgekühlt und mit Clarcel (3,2 kg) versetzt. Mit verdünnter Salzsäure (36%-ig, 0,8 kg in etwa 440 L Wasser) wird auf einen pH von etwa 2,0 eingestellt. Die erhaltene Lösung wird filtriert und mit Methylenchlorid (58 L) extrahiert. Die Methylenchloridphase wird verworfen. Zur wäßrigen Phase wird erneut Methylenchlorid (130 L) gegeben und mit einer vorbereiteten Soda-Lösung (11,0 kg in 51 L Wasser) wird ein pH von etwa 10,0 eingestellt. Die Methylenchlorid-Phase wird abgetrennt und die wäßrige Phase mit Methylenchlorid (136 L) extrahiert. Im schwachen Vakuum (600 - 700 mbar) wird bei 40°C von den vereinigten Methylenchloridphasen Methylenchlorid (etwa 175 L) abdestilliert. Der Apparateinhalt wird auf 20°C abgekühlt, Acetylchlorid (etwa 0,5 kg) wird zugesetzt und es wird für etwa 40 Minuten bei 20°C gerührt. Die Reaktionslösung wird in einen zweiten Apparat überführt. Mit einer vorbereiteten Salzsäure-Lösung (4,7 kg Salzsäure 36 %ig in 460 L Wasser) wird bei 20°C ein pH von 2,0 eingestellt. Die Methylenchlorid-Phase wird abgetrennt und entsorgt. Die wäßrige Phase wird mit Methylenchlorid (39 L) gewaschen. Dann wird Methylenchlorid (130 L) zugesetzt und mit einer vorbereiteten Soda-Lösung (7,8 kg Soda in 38 L Wasser) bei 20°C ein pH von 10,0 eingestellt. Nach 15 Min. Rühren wird die organische Phase abgetrennt und die wäßrige Phase zweimal mit Methylenchlorid (97 L und 65 L) gewaschen. Die Methylenchlorid-Phasen werden vereinigt und im schwachen Vakuum ein Teil des Methylenchlorids (90 L) bei einer Temperatur von 30 - 40°C abdestilliert. Anschließend wird Dimethylformamid (114 kg) zugesetzt und unter Vakuum bei 40°C das restliche Methylenchlorid abdestilliert. Der Apparateinhalt wird auf 20°C abgekühlt.

### Darstellung des Tiotropiumbromids (I)

Zur nach vorhergehender Vorschrift erhältlichen Scopinester-Lösung wird bei 20°C Methylbromid (5,1 kg) eingeleitet. Der Apparateinhalt wird bei 30°C für etwa 2,5 Tage gerührt. Bei 50°C werden im Vakuum 70 L DMF abdestilliert. Die Lösung wird in einen kleineren Apparat überführt. Es wird mit DMF (10 L) nachgespült.. Bei 50°C wird im Vakuum weiteres DMF abdestilliert bis eine Gesamtdestillatmenge von etwa 100 L erreicht ist. Es wird auf 15°C abgekühlt und bei dieser Temperatur 2 h nachgerührt. Das Produkt wird mittels Nutschentrockner isoliert, mit 15°C kaltem DMF (10 L) und 15°C kaltem Aceton (25 L) gewaschen. Es wird bei max. 50°C für max. 36 Std. im Stickstoffstrom getrocknet. Ausbeute: 13,2 kg (88 %);
Schmp.: 200-230°C (abh. von Reinheitsgrad des Rohprodukts);

Das so erhaltene Rohprodukt (10,3 kg) wird in Methanol (66 L) eingetragen. Das Gemisch wird zum Lösen zum Rückfluß erhitzt. Die Lösung wird auf 7°C abgekühlt und 1,5 h bei dieser Temperatur nachgerührt. Das Produkt wird mittels Nutschentrockner isoliert, mit 7°C kaltem Methanol (11 L) gewaschen und max. 36 h bei etwa 50°C im Stickstoffstrom getrocknet. Ausbeute: 9,9 kg (96 %);
Schmp.: 228°C (bestimmt über DSC bei Heizrate von 10K/min).

Gegebenenfalls kann das so erhaltene Produkt in das kristalline Monohydrat desTiotropiumbromids überführt werden. Hierzu kann wie folgt vorgegangen werden. In einem geeigneten Reaktionsgefäß werden in 25,7 kg Wasser 15,0 kg Tiotropiumbromid eingetragen. Die Mischung wird auf 80-90°C erhitzt und bei gleichbleibender Temperatur solange gerührt, bis eine klare Lösung entsteht. Aktivkohle (0,8 kg), wasserfeucht, wird in 4,4 kg Wasser aufgeschlämmt, diese Mischung in die Tiotropiumbromid-haltige Lösung eingetragen und mit 4,3 kg Wasser nachgespült. Die so erhaltene Mischung wird wenigstens 15 min bei 80-90°C gerührt und anschließend über einen beheizten Filter in einen auf 70°C Manteltemperatur vorgewärmten Apparat filtriert. Der Filter wird mit 8,6 kg Wasser nachgespült. Der Apparateinhalt wird mit 3-5°C pro 20 Minuten auf eine Temperatur von 20-25°C abgekühlt. Mit Kaltwasserkühlung wird der Apparat auf 10-15°C weiter abgekühlt und die Kristallisation durch mindestens einstündiges Nachrühren vervollständigt. Das Kristallisat wird über einen Nutschentrockner isoliert, der isolierte Kristallbrei mit 9 L kaltem Wasser (10-15°C) und kaltem Aceton (10-15°C) gewaschen. Die erhaltenen Kristalle werden bei etwa 25°C über etwa 2 Stunden im Stickstoffstrom getrocknet. Ausbeute : 13,4 kg Tiotropiumbromid-monohydrat (86 % d. Th.)
Schmp.: 230°C (bestimmt über DSC bei Heizrate von 10K/min).

## Patentansprüche

1. Verfahren zur Herstellung von Tiotropiumbromid der Formel (I) 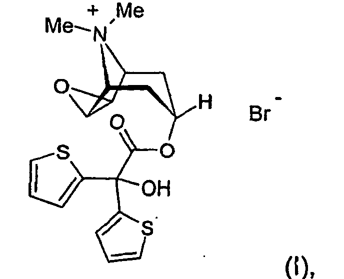 **dadurch gekennzeichnet, daß** der Tropenolester der Formel (VII) 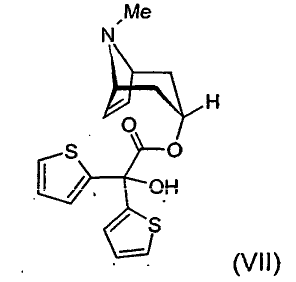 zum Scopinester der Formel (IV) 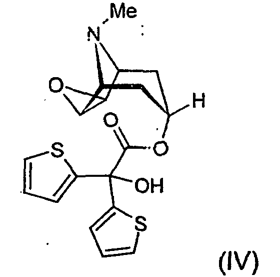 epoxidiert wird und dieser mittels Methylbromid zu Tiotropiumbromid (I) quartemiert wird.

2. Verfahren nach Anspruch 1, **dadurch gekennzeichnet, daß** zur Epoxidation von (VII) zu (IV) als Epoxidierungsmittel ein Gemisch von Vanadiumpentoxid mit Wasserstoffperoxid Verwendung findet.

3. Verfahren nach Anspruch 1 oder 2, **dadurch gekennzeichnet, daß** die Epoxidation von (VII) zu (IV) in einem Lösemittel durchgeführt wird, welches ausgewählt ist aus der Gruppe bestehend aus Wasser, Dimethylfomamid, Acetonitril, Dimethylacetamid und N-Methylpyrrolidinon.

4. Verfahren nach einem der Ansprüche 1, 2 oder 3, **dadurch gekennzeichnet, daß** die Quarternierung von (IV) zu (I) mittels Methylbromid in einem Lösemittel durchgeführt wird, welches ausgewählt ist aus der Gruppe bestehend aus Wasser, Dimethylfomamid, Dimethylacetamid, N-Methylpyrrolidinon und Dichlormethan.

5. Verfahren nach einem der Ansprüche 1 bis 4, **dadurch gekennzeichnet, daß** die Verbindung der Formel (VII) 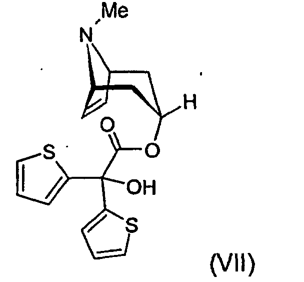 durch Umsetzung von Tropenol der Formel (V), 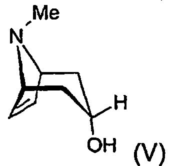 gegebenenfalls in Form seiner Säureadditionssalze, mit einem Ester der Formel (VI) 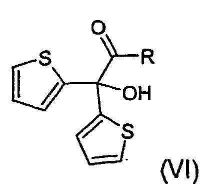 in dem R ein Rest ist, der ausgewählt ist aus der Gruppe bestehend aus Hydroxy, Methoxy, Ethoxy, O-N-Succinimid, O-N-Phthalimid, Phenyloxy, Nitrophenyloxy, Fluorophenyloxy, Pentafluorophenyloxy, Vinyloxy, -S-Methyl, -S-Ethyl und -S-Phenyl, erhalten wird.

6. Verfahren nach Anspruch 5, **dadurch gekennzeichnet, daß** das Tropenol (V) in Form eines Säureadditionssalzes eingesetzt wird, welches ausgewählt ist aus der Gruppe bestehend aus Hydrochlorid, Hydrobromid, Hydrogenphosphat, Hydrogensulfat, Tetrafluoroborat und Hexafluorophosphat.

7. Verfahren nach einem der Ansprüche 5 oder 6, **dadurch gekennzeichnet, daß** die Umsetzung von (V) zu (VII) in einem geeigneten organischen Lösemittel durchgeführt wird, vorzugsweise in einem Lösemittel. welches ausgewählt ist aus der Gruppe bestehend aus Toluol, Benzol, n-Buthylacetat, Dichlormethan, THF, Dioxan, Dimethylacetamid, DMF und N-Methylpyrrolidinon.

8. Verfahren nach einem der Ansprüche 5, 6 oder 7, **dadurch gekennzeichnet, daß** die Umsetzung von (V) zu (VII) in Gegenwart einer organischen oder anorganischen Base durchgefürt wird, vorzugsweise in Gegenwart einer Base, die ausgewählt ist aus der Gruppe bestehend aus organischen Aminen, besonders bevorzugt Diisopropylethylamin, Triethylamin, DBU oder Pyridin, oder aus der Gruppe von anorganischen Basen, bestehend aus den Alkalioder den Erdalkalicarbonaten, Alkoholaten und Hydriden des Lithiums, Natriums, Kaliums und Calciums.

9. Verfahren nach einem der Ansprüche 5, 6 oder 7, **dadurch gekennzeichnet, daß** in dem Fall, daß in der Verbindung der Formel (VI) R Hydroxy bedeutet, die Umsetzung von (V) zu (VII) in Gegenwart von Kopplungsreagenzien durchgeführt wird, die bevorzugt ausgewählt sind aus der Gruppe bestehend aus Carbonyldiimidazol, Carbonyldi-1,2,4-triazol, Dicyclohexylcarbodiimid und Ethyl-dimethylaminopropylcarbodiimid.

10. Verwendung von Tropenol der Formel (V), 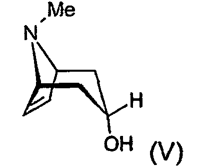 gegebenenfalls in Form seiner Säureadditionssalze, als Ausgangsmaterial zur Herstellung von Tiotropiumbromid der Formel (I)

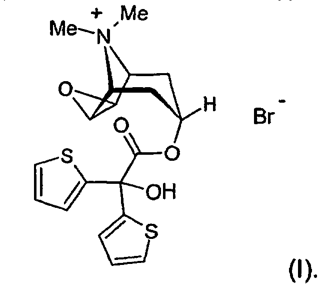

11. Verwendung von Tropenolester der Formel (VII) 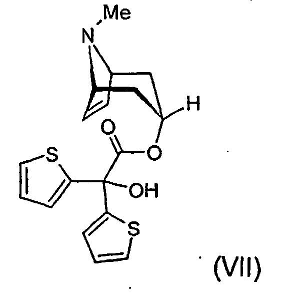 gegebenenfalls in Form seiner Säureadditionssalze, als Ausgangsmaterial zur Herstellung von Tiotropiumbromid der Formel (I)

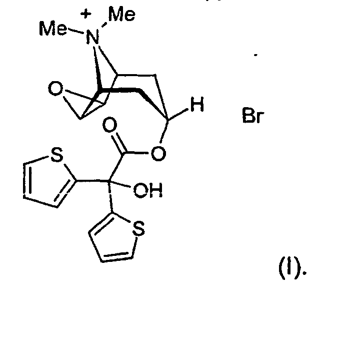

## Claims

1. Process for preparing tiotropium bromide of formula (I) 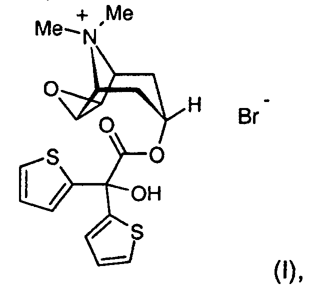 **characterised in that** the tropenol ester of formula (VII) 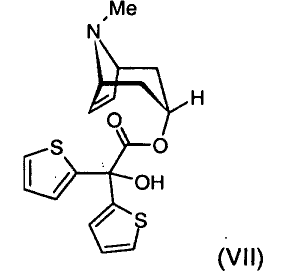 is epoxidised to form the scopine ester of formula (IV) 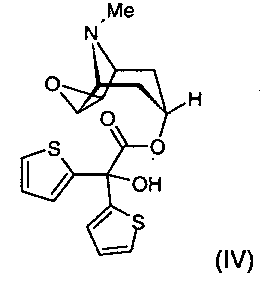 which is then quaternised with methyl bromide to form tiotropium bromide (I).

2. Process according to claim 1, **characterised in that** a mixture of vanadium pentoxide with hydrogen peroxide is used as the epoxidising agent for epoxidising (VII) to form (IV).

3. Process according to claim 1 or 2, **characterised in that** the epoxidation of (VII) to form (IV) is carried out in a solvent which is selected from among water, dimethylformamide, acetonitrile, dimethylacetamide and N-methylpyrrolidinone.

4. Process according to one of claims 1, 2 or 3, **characterised in that** the quaternisation of (IV) to form (I) is carried out using methylbromide in a solvent which is selected from among water, dimethylformamide, dimethylacetamide, N-methylpyrrolidinone and dichloromethane.

5. Process according to one of claims 1 to 4, **characterised in that** the compound of formula (VII) 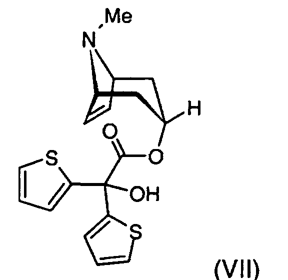 is obtained by reacting tropenol of formula (V), 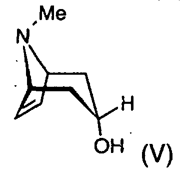 optionally in the form of the acid addition salts thereof, with an ester of formula (VI) 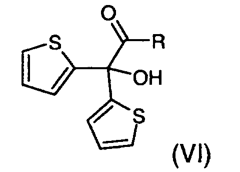 in which R is a group which is selected from among hydroxy, methoxy, ethoxy, O-N-succinimide, O-N-phthalimide, phenyloxy, nitrophenyloxy, fluorophenyloxy, pentafluorophenyloxy, vinyloxy, -S-methyl, -S-ethyl and -S-phenyl.

6. Process according to claim 5, **characterised in that** the tropenol (V) is used in the form of an acid addition salt thereof which is selected from among the hydrochloride, hydrobromide, hydrogen phosphate, hydrogen sulphate, tetrafluoroborate and hexafluorophosphate.

7. Process according to one of claims 5 or 6, **characterised in that** the reaction of (V) to form (VII) is carried out in a suitable organic solvent, preferably in a solvent which is selected from among toluene, benzene, n-butylacetate, dichloromethane, THF, dioxane, dimethylacetamide, DMF and N-methylpyrrolidinone.

8. Process according to one of claims 5, 6 or 7, **characterised in that** the reaction of (V) to form (VII) is carried out in the presence of an organic or inorganic base, preferably in the presence of a base selected from among the organic amines, most preferably diisopropylethylamine, triethylamine, DBU or pyridine, or from among the inorganic bases, comprising the alkali metal or alkaline earth metal carbonates, alkoxides and hydrides of lithium, sodium, potassium and calcium.

9. Process according to one of claims 5, 6 or 7, **characterised in that**, if R denotes hydroxy in the compound of formula (VI), the reaction of (V) to form (VII) is carried out in the presence of coupling reagents which are preferably selected from among carbonyldiimidazole, carbonyldi-1,2,4-triazole, dicyclohexylcarbodiimide and ethyl-dimethylaminopropylcarbodiimide.

10. Use of tropenol of formula (V), 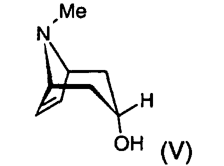 optionally in the form of the acid addition salts thereof, as a starting material for the preparation of tiotropium bromide of formula (I)

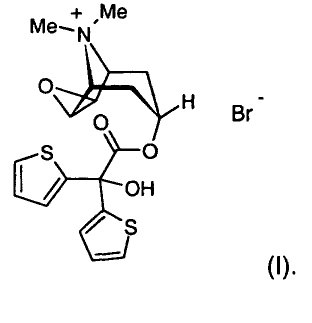

11. Use of tropenol ester of formula (VII) 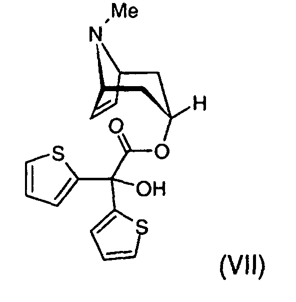 optionally in the form of the acid addition salts thereof, as a starting material for the preparation of tiotropium bromide of formula (I)

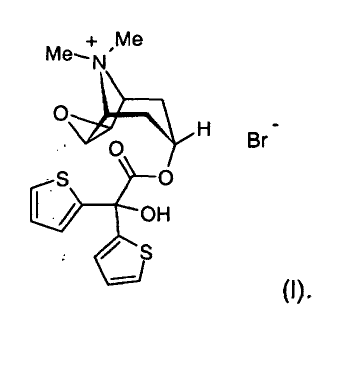

## Revendications

1. Procédé de production du bromure de tiotropium de formule (I) 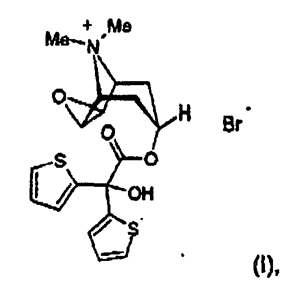 **caractérisé en ce que** l'ester de tropénol de formule (VII) 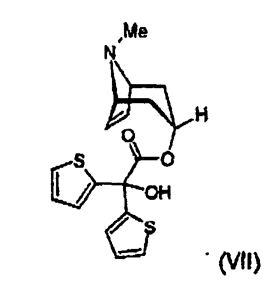 est époxydé en l'ester de scopine de formule (IV) 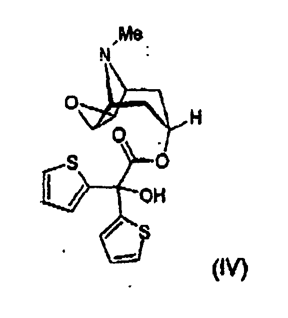 et celui-ci est quaternisé en le bromure de tiotropium (I) avec le bromure de méthyle.

2. Procédé selon la revendication 1 **caractérisé en ce qu'**un mélange de pentoxyde de vanadium et de peroxyde d'hydrogène est utilisé comme agent d'époxydation pour l'époxydation de (VII) en (IV).

3. Procédé selon la revendication 1 ou 2 **caractérisé en ce que** l'époxydation de (VII) en (IV) est conduite dans un solvant qui est choisi dans le groupe consistant en l'eau, le diméthylformamide, l'acétonitrile, le diméthylacétamide et la N-méthylpyrrolidinone.

4. Procédé selon l'une des revendications 1, 2 et 3 **caractérisé en ce que** la quaternisation de (IV) en (I) avec le bromure de méthyle est conduite dans un solvant qui est choisi dans le groupe consistant en l'eau, le diméthylformamide, le diméthylacétamide, la N-méthylpyrrolidinone et le dichlorométhane.

5. Procédé selon l'une des revendications 1 à 4 **caractérisé en ce que** le composé de formule (VII) 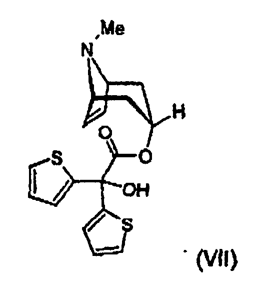 est obtenu par réaction du tropénol de formule (V) 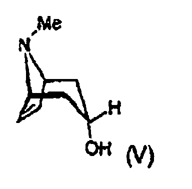 éventuellement sous forme de ses sels d'addition d'acide, avec un ester de formule (VI) 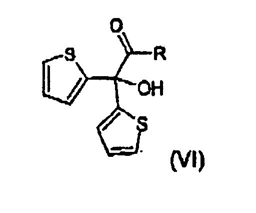 où R est un reste qui est choisi dans le groupe consistant en hydroxyle, méthoxy, éthoxy, O-N-succinimide, O-N-phtalimide, phényloxy, nitrophényloxy, fluoro-phényloxy, pentafluorophényloxy, vinyloxy, -S-méthyle, -S-éthyle et -S-phényle.

6. Procédé selon la revendication 5 **caractérisé en ce que** le tropénol (V) est utilisé sous forme d'un sel d'addition d'acide qui est choisi dans le groupe consistant en le chlorhydrate, le bromhydrate, l'hydrogénophosphate, l'hydrogénosulfate, le tétrafluoroborate et l'hexafluorophosphate.

7. Procédé selon l'une des revendications 5 et 6 **caractérisé en ce que** la conversion de (V) en (VII) est conduite dans un solvant organique approprié, de préférence dans un solvant qui est choisi dans le groupe consistant en le toluène, le benzène, l'acétate de n-butyle, le dichlorométhane, le THF, le dioxane, le diméthylacétamide, le DMF et la N-méthylpyrrolidinone.

8. Procédé selon l'une des revendications 5, 6 et 7 **caractérisé en ce que** la conversion de (V) en (VII) est conduite en présence d'une base organique ou inorganique, de préférence en présence d'une base qui est choisie dans le groupe consistant en les amines organiques, de manière particulièrement préférée la diisopropyléthylamine, la triéthylamine, DBU ou la pyridine, ou dans le groupe des bases inorganiques consistant en les carbonates alcalins ou alcalino-terreux, les alcoolates et les hydrures de lithium, de sodium, de potassium ou de calcium.

9. Procédé selon l'une des revendications 5, 6 et 7 **caractérisé en ce que**, dans le cas où R représente hydroxyle dans le composé de formule (VI), la conversion de (V) en (VII) est conduite en présence de réactifs de couplage qui sont choisis de préférence dans le groupe consistant en le carbonyldiimidazole, le carbonyldi-1,2,4-triazole, le dicyclohexylcarbodiimide et l'éthyl-diméthylaminopropylcarbodiimide.

10. Utilisation du tropénol de formule (V) 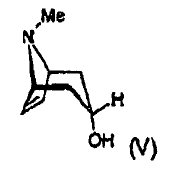 éventuellement sous forme de ses sels d'addition d'acide, comme produit de départ pour la production du bromure de tiotropium de formule (I)

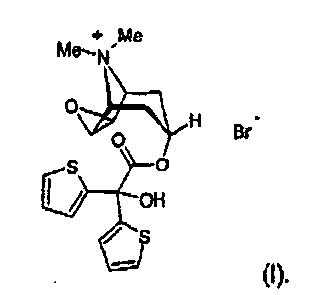

11. Utilisation de l'ester de tropénol de formule (VII) 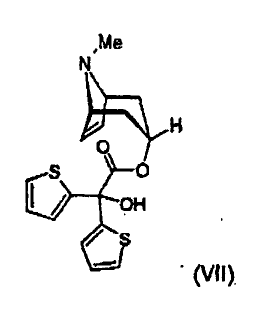 éventuellement sous forme de ses sels d'addition d'acide, comme produit de départ pour la production du bromure de tiotropium de formule (I)
